# FASCICULE DE BREVET EUROPEEN

(11) **EP 2 525 770 B1**
(45) Date de publication et mention de la délivrance du brevet: **13.06.2018**
(21) Numéro de dépôt: 11704663.1
(22) Date de dépôt: 20.01.2011
(51) Int. Cl.: A61K 6/00, A61K 6/097

(54) **MATERIAU D'INSERTION DESTINE A ELARGIR LE SILLON GINGIVAL**
EINSETZMATERIAL ZUR ERWEITERUNG DES ZAHNFLEISCHSULCUS
INSERTION MATERIAL INTENDED FOR WIDENING THE GINGIVAL CREVICE

(30) Priorité: 21.01.2010 FR 1050395
(43) Date de publication de la demande: 28.11.2012
(73) Titulaire: Lesage, Patrick, 35400 Saint Malo (FR)
(72) Inventeur: Lesage, Patrick, 35400 Saint Malo (FR)
(74) Mandataire: Hubert, Philippe
(86) Numéro de dépôt international: PCT/FR2011/050106
(87) Numéro de publication internationale: WO 2011/089362

(56) Documents cités:
- EP-A1- 2 036 533
- WO-A1-90/15587
- WO-A2-2008/021740
- DE-A1- 3 736 155
- US-A1- 2005 287 494

## Description

La présente invention a pour objet un nouveau matériau d'insertion utile pour élargir le sillon gingival, sensiblement sans saignement. Elle trouve application dans le domaine dentaire.

Le sillon gingival est un espace virtuel, se situant entre la dent et la gencive, qu'il est nécessaire d'élargir pour la réalisation de soins dentaires divers, par exemple préalablement à la prise d'une empreinte.

D'une façon générale, l'élargissement du sillon gingival peut être obtenu :
- soit par éviction, par exemple à l'aide d'un bistouri électrique ou d'une fraise diamantée ;
- soit par rétraction, en insérant dans le sillon gingival un matériau préformé, tel qu'un cordonnet de coton imbibé ou non d'une solution destinée à favoriser la rétraction, ou une composition injectable susceptible de durcir par réaction chimique ou par gonflement physique.

Le brevet EP 0477 244 décrit un matériau d'insertion d'une conception originale se présentant sous la forme d'une pate biocompatible et injectable comprenant essentiellement :
- un agent destiné à absorber les fluides, tel qu'en particulier de l'argile kaolinique (ou argile blanche) ou une farine d'algues ; de l'eau ;
- un agent astringent, de préférence le chlorure d'aluminium, destiné à faciliter l'arrêt des saignements par son action de rétraction en conduisant à une bonne hémostase de surface.

Ce brevet a conduit à la mise au point, puis à la commercialisation du produit dénommé Expasyl® qui a ouvert une nouvelle voie de traitement dans toutes les indications d'ouverture sulculaire temporaire.

Ce produit est en effet particulièrement avantageux, notamment par comparaison aux matériaux durcissables par réaction chimique, en raison de sa simplicité d'utilisation (absence de mélange préalable) et de sa rapidité d'action (pas de temps de prise).

En outre, contenant du chlorure d'aluminium, le produit Expasyl® conduit à une ouverture du sulcus avec une excellente hémostase.

Cependant, le produit Expasyl® présente un certain nombre d'inconvénients. Tout d'abord, en raison du caractère très hydrophile et hydrosoluble de l'argile kaolinique, ce produit doit être maintenu à l'abri de la salive pour éviter sa dilution prématurée créant ainsi une situation d'inconfort pour le patient qui doit éviter de fermer la bouche pendant son utilisation.

Pour les mêmes raisons, une fois mis en place, ce produit ne peut être utilisé conjointement avec une instrumentation comportant un spray d'eau.

Quelques soient les précautions prises, on a constaté que le produit Expasyl® est dilué par la salive et les fluides sulculaires en perdant sa consistance et son efficacité au bout d'environ deux minutes limitant d'autant le temps du travail du dentiste.

Par ailleurs, l'élimination de ce produit après utilisation est relativement longue et nécessite une grande quantité d'eau notamment pour éviter tout dépôt de kaolin sur la dentine qui peut interférer par exemple avec des collages, ainsi que pour éliminer le chlorure qui peut interférer avec la prise des matériaux à empreinte de la classe des polyéthers.

L'utilisation d'une grande quantité d'eau entraîne le délitement du produit et la libération de ses éléments constitutifs et en particulier du chlorure d'aluminium. Or, le chlorure d'aluminium présente un goût particulièrement désagréable et persistant et peut entraîner une irritation des muqueuses.

On connaît également par le document WO 2007/128926 un matériau utilisable pour former un pansement sur les muqueuses buccales ou sur la peau constitué d'une pâte biocompatible contenant, à titre de constituant essentiel, du kaolin naturel, un agent humectant et un agent de formation d'hydrogel, de préférence la silice colloïdale. Ce matériau contient entre 35 et 55 % en masse de kaolin et se délite très rapidement en présence d'eau. De ce fait, il présente les mêmes inconvénients que le produit Expasyl®. De plus, ce matériau ne permet pas l'ouverture du sulcus. Il n'est donc pas utilisable dans les applications envisagées par la présente invention.

Le document US-A-20057287494 divulgue des compositions de rétraction dentaire sous forme d'une pâte comprenant un agent astringent, des fibres polymèriques comme agents de texture et de l'eau.

Dans ce contexte, la présente invention a pour but de résoudre le problème technique consistant en la fourniture d'un nouveau matériau d'insertion présentant les avantages du produit Expasyl® sans souffrir des mêmes inconvénients.

Il a été découvert, et ceci constitue le fondement de la présente invention, qu'il était possible de résoudre ce problème technique d'une façon entièrement satisfaisante et susceptible d'être mise en oeuvre de façon aisée à l'échelle industrielle en incorporant l'agent astringent dans une matrice permettant une libération lente de l'agent astringent et présentant une cohésion suffisante en milieu aqueux pour éviter la perte de ses propriétés mécaniques pendant une durée compatible avec tout type de traitement.

Ainsi, selon un premier aspect, la présente invention a pour objet un matériau d'insertion destiné à élargir le sillon gingival, caractérisé en ce qu'il est constitué d'une pâte hydrophile comprenant, exprimé en pourcentage en masse rapportée à la masse totale du matériau :
- entre 5 et 15 %, de préférence entre 6 et 12 % d'un agent astringent ;
- entre 25 et 50 %, de préférence entre 30 et 45 % d'un agent de texture choisi parmi :
   - les biopolymères hydrophiles ;
   - les biopolymères hydrophiles associés à des fibres végétales ;
   - les biopolymères hydrophiles associés à des fibres végétales et à de l'alcool polyvinylique ;
   - les fibres végétales associées à de l'alcool polyvinylique ;
- entre 30 et 60 %, de préférence entre 30 et 55 % d'eau ; et
- entre 0 et 20 %, de préférence entre 5 et 18 % d'un agent humectant ;
   - la quantité totale d'eau et d'agent humectant étant comprise entre 35 et 65 %, de préférence entre 40 et 60 % en masse rapportée à la masse totale du matériau ;
   - les biopolymères hydrophiles précités étant choisis dans le groupe constitué par les pectines ; les galactomannanes des graines de guar, de caroube, de tara ; le scléroglucane ; le xanthane et les carraghénanes, de préférence de type iota, et leurs mélanges ;
   - les fibres végétales précitées étant choisies parmi les fibres de pois et les fibres d'avoine ;
   - l'agent astringent étant choisi dans le groupe constitué des chlorures et sulfates de fer ou d'aluminium, du sulfate double d'aluminium et de potassium, et de leurs mélanges ;
   - ledit matériau d'insertion présentant le profil de libération suivant de l'agent astringent, mesuré selon la méthode de dissolution décrite dans la Pharmacopée Européenne, 6^{ème} édition (Ph. Eur. 2.9.3 (01/2008) à 150 t.p.m dans 700 ml d'eau purifiée à 37°C :
- moins de 20 % d'agent astringent libéré après 5 mn ;
- moins de 40 % d'agent astringent libéré après 10 mn ;
- moins de 50 % d'agent astringent libéré après 15 mn ;
- moins de 60 % d'agent astringent libéré après 30 mn ;
- moins de 80 % d'agent astringent libéré après 60 mn.

Comme on le comprend, l'originalité du matériau conforme à la présente invention réside en premier lieu dans la nature de l'agent de texture formant matrice, qui le compose.

Cet agent de texture peut être constitué :
- soit, d'un ou plusieurs biopolymères hydrophiles ;
- soit, d'un ou plusieurs biopolymères hydrophiles associé(s) à des fibres végétales ;
- soit, d'un ou plusieurs biopolymères hydrophiles associé(s) à des fibres végétales et à de l'alcool polyvinylique ;
- soit, à des fibres végétales associées à de l'alcool polyvinylique.

Dans le matériau d'insertion conforme à l'invention, l'agent de texture représente entre 25 et 50 %, de préférence entre 30 et 45 % en masse, rapporté à la masse totale du matériau.

Les biopolymères hydrophiles susceptibles d'être utilisés dans le cadre de la présente invention sont avantageusement des polysaccharides d'origine naturelle, de préférence de qualité alimentaire, présentant un pouvoir gélifiant ou épaississant, choisis dans le groupe constitué par les pectines ; les galactomannanes des graines de guar, de caroube, de tara ; le scléroglucane ; le xanthane ; les carraghénanes et leurs mélanges.

Ce biopolymère est avantageusement choisi pour obtenir une matrice présentant des propriétés rhéologiques stables dans le temps. A cet effet, l'agent de texture conforme à l'invention est exempt ou ne contient qu'une quantité faible, inférieure à 10 % en poids rapportée au poids de l'agent de texture, d'alginates.

En effet, il a été observé que les gels susceptibles d'être formés à partir des alginates sont irréversibles et ne présentent pas une consistance stable dans le temps compatible avec leur mise en oeuvre dans le cadre de l'invention.

De préférence, le biopolymère hydrophile sera choisi parmi les carraghénanes.

Les carraghénanes sont généralement obtenus par extraction à chaud d'algues rouges de la famille des Rhodophyceae, en particulier de Chondrus et Eucheuma.

Ce sont des copolymères d'éther sulfate de D-galactose et de 3,6-anhydro-D-galactose qui peuvent se présenter sous forme de sels, en particulier de potassium ou de calcium.

Avantageusement, les carraghénanes utilisées dans le cadre de l'invention sont de type iota, comme en particulier les produits commercialisés par la société FMC BIOPOLYMERE sous les dénominations Viscarin® ou Gelcarin®.

D'excellents résultats ont été obtenus dans le cadre de la présente invention avec le produit Viscarin® PC 389.

Lorsque l'agent de texture est exclusivement constitué d'un ou plusieurs biopolymères hydrophiles, il représente avantageusement 40 à 50 % en masse, et de préférence encore environ 45 % en masse rapportée à la masse totale du matériau d'insertion.

Les fibres végétales susceptibles d'être utilisées dans le cadre de la présente invention sont choisies parmi les fibres de pois et les fibres d'avoine.

De tels produits sont bien connus dans le domaine cosmétique et sont, par exemple, commercialisés par la société Beacon CMP sous les dénominations Tech-O®.

Dans le cadre de la présente invention, les fibres végétales sont utilisées soit en association avec un ou plusieurs polymères hydrophiles, soit en association avec de l'alcool polyvinylique, soit encore en association avec un ou plusieurs biopolymères hydrophiles et de l'alcool polyvinylique.

D'une façon générale, le rapport pondéral entre les fibres végétales et les biopolymères hydrophiles est compris entre 1:1 et 1,5:1, de préférence de l'ordre de 1,25, tandis que le rapport pondéral entre les fibres végétales et l'alcool polyvinylique est généralement compris entre 12:1 et 20:1, de préférence de l'ordre de 15:1.

L'alcool polyvinylique susceptible d'être utilisé dans le cadre de la présente invention est de préférence de qualité cosmétique ou pharmaceutique.

Avantageusement, ce produit doit présenter une viscosité relativement élevée, par exemple de l'ordre d'au moins 20 mPa.s en solution aqueuse à 4 %.

Parmi les alcools polyvinyliques susceptibles d'être utilisés dans le cadre de la présente invention, on peut citer les produits commercialisés sous la dénomination Gohsenol® EG, comme en particulier les produits Gohsenol® EG-25, Gohsenol® EG-30 et Gohsenol® EG-40.

L'alcool polyvinylique est généralement utilisé :
- en une quantité comprise entre 1 et 3 % en masse, de préférence 1,5 à 3 % en masse, et de préférence encore d'environ 2 % en masse rapporté à la masse totale du matériau lorsqu'il est associé à des fibres végétales ; et
- en une quantité comprise entre 0,5 et 2 % en masse, de préférence de l'ordre de 1 % en masse lorsqu'il est utilisé en association avec des fibres végétales et un ou plusieurs biopolymères hydrophiles.

L'agent astringent susceptible d'être utilisé dans le cadre de la présente invention est généralement choisi dans le groupe constitué des chlorures et sulfates de fer ou d'aluminium, du sulfate double d'aluminium et de potassium, et de leurs mélanges.

Dans le mode de réalisation actuellement préféré de l'invention, l'agent astringent est le chlorure d'aluminium et peut être utilisé en une quantité avantageusement comprise entre 7 et 10 % en masse, rapportée à la masse totale du matériau d'insertion.

La quantité d'eau contenue dans le matériau d'insertion conforme à l'invention peut varier notamment en fonction de la nature de l'agent de texture et de son association ou non avec un agent humectant.

L'eau, de préférence purifiée, est utilisée en une quantité suffisante pour obtenir une pâte cohésive présentant les caractéristiques physico-chimiques qui seront détaillées ultérieurement.

De façon générale, l'eau est présente au sein du matériau d'insertion en une quantité comprise entre 30 et 60 % en masse, de préférence entre 30 et 55 % en masse, rapportée à la masse totale du matériau.

L'agent humectant susceptible d'être utilisé dans le cadre de la présente invention est généralement choisi dans le groupe constitué par le glycérol, le polyéthylèneglycol, le sorbitol et leurs mélanges. Il s'agira de préférence de glycérol (ou glycine).

L'agent humectant ne constitue pas un composant essentiel du matériau d'insertion selon l'invention. Il est avantageusement utilisé pour améliorer la stabilité du matériau d'insertion notamment pour éviter le dessèchement de ce matériau lors de son stockage.

Lorsqu'il est utilisé, l'agent humectant est présent au sein du matériau en une quantité généralement comprise entre 5 et 18 % en masse, de préférence entre 8 et 16 % en masse, rapportée à la masse totale du matériau.

Le matériau d'insertion conforme à l'invention est exempt de kaolin, à tout le moins en proportion significative, par exemple supérieure à 10 % en masse rapportée à la masse totale du matériau.

Le matériau d'insertion conforme à la présente invention peut également comprendre jusqu'à 1 % en masse, et de préférence de 0,01 à 0,5 % en masse d'au moins un additif choisi parmi les colorants et les arômes, de préférence de qualité alimentaire.

Tout type de colorant peut être utilisé dans le cadre de la présente invention.

S'agissant d'applications dentaires, on utilisera de préférence un colorant d'une couleur qui tranche avec la couleur de la dent et de la gencive, comme par exemple le bleu, le jaune ou le vert.

Le colorant peut être utilisé en une quantité généralement comprise entre 0,005 % et 0,05 %, de préférence d'environ 0,01 % en masse, rapportée à la masse totale du matériau.

Il n'y a pas non plus de limitation quant à la nature de l'arôme susceptible d'être utilisé dans le cadre de la présente invention. Il peut s'agir d'un arôme d'amande, de menthe ou de citron par exemple.

La quantité d'arôme qui peut être utilisé est généralement comprise entre 0,1 et 1 %, et de préférence d'environ 0,5 % en masse, rapportée à la masse totale du matériau.

Le matériau d'insertion conforme à l'invention peut, en outre, comprendre jusqu'à 10 % en masse, et de préférence de 2 à 10 % en masse, d'au moins une substance active choisie parmi les anesthésiques locaux.

Parmi les anesthésiques locaux susceptibles d'être utilisés dans le cadre de l'invention, on peut citer la lidocaïne, la prilocaïne, la bupivacaïne, la benzoïne, la tétracaïne, la mépivacaïne et la ropivacaïne.

Avantageusement, l'anesthésique local est la lidocaïne et peut être utilisée en une quantité d'environ 2 à 7 % en masse, rapportée à la masse totale du matériau.

Le matériau d'insertion conforme à la présente invention présente des propriétés physicochimiques originales qui lui permettent de satisfaire aux exigences de son utilisation dans le domaine dentaire, notamment pour élargir le sillon gingival.

Ainsi, ce matériau présente, en premier lieu, et ceci constitue une caractéristique technique originale de la présente invention, un profil spécifique de dissolution en milieu aqueux qui peut être mesuré par la libération spécifique de l'agent astringent.

Plus précisément, la quantité d'agents astringents libérés, mesurée selon la méthode de dissolution décrite dans la pharmacopée européenne, 6^{ème} édition (Ph. Eeur. 2.9.3 (01/2008)) à 150 tr/min dans 700 ml d'eau purifiée à 37°C est de :
- moins de 20 % après 5 min ;
- moins de 40 %, et de préférence moins de 30 % après 10 min ;
- moins de 50 %, et de préférence moins de 40 % après 15 min ;
- moins de 60 %, et de préférence moins de 55 % après 30 min ;
- moins de 80 %, et de préférence moins de 70 % après 60 min.

La quantité d'agent astringent libéré peut être déterminée par des méthodes de dosage bien connues de l'homme du métier.

Dans le cas du chlorure d'aluminium, qui constitue l'agent astringent actuellement préféré, ce dosage peut être réalisé par titrage complexométrique, par exemple selon la méthode décrite dans la pharmacopée européenne, 6ème édition (01/2008) au chapitre 2.5.11.

Il a été montré que le matériau d'insertion conforme à la présente invention libère l'agent astringent beaucoup plus lentement que le produit Expasyl®, et permet donc une utilisation prolongée en bouche.

A titre d'exemple, la quantité d'agent astringent libéré après 10 min avec le produit Expasyl® est d'environ 55 % alors que cette même quantité est inférieure à 40 %, voire inférieure à 30 % avec un matériau selon l'invention.

Par ailleurs, le matériau d'insertion conforme à la présente invention présente une excellente cohésion dans le temps. Ainsi, ce matériau ne présente aucune désagrégation visible au bout de 5 h selon la méthode de désagrégation des comprimés et des capsules décrites dans la pharmacopée européenne, 6ème édition (01/2008) au chapitre 2.9.1, pour un échantillon présentant un diamètre de 10 mm et une épaisseur de 4 mm et un milieu de désagrégation constitué d'eau purifiée à 37°C.

Dans le même test, on a constaté que le matériau Expasyl® se désagrège totalement au bout de 15 min.

Enfin, des essais d'extrusion, de compression et de cisaillement ont montré que le matériau d'insertion selon l'invention présente l'une au moins des propriétés mécaniques suivantes :
- un comportement rhéologique essentiellement plastique ;
- un comportement tribologique de type glissant avec une friction seuil, de préférence comprise entre 10 et 60 kPa ;
- un seuil de cisaillement initial compris entre 30 et 350 kPa ;
- un module de Young compris entre 65 et 200 kPa.

Le matériau d'insertion conforme à la présente invention peut être fabriqué facilement par simple mélange de ses constituants.

De façon générale, un matériau d'insertion selon l'invention peut être obtenu en mélangeant :
- d'une part, un mélange contenant de l'eau purifiée et l'agent astringent et éventuellement un agent humectant comme le glycérol et un colorant tel que le bleu patenté ; et
- d'autre part, un mélange contenant un agent de texture tel qu'un polysaccharide, de préférence de type carraghénane iota, éventuellement associé à une fibre végétale et/ou à de l'alcool polyvinylique.

L'invention sera mieux comprise à la lecture des exemples non limitatifs suivants.

### EXEMPLE 1

Etape 1: Dans un bécher de 40 l en polyéthylène basse densité, 16,758 litres d'eau purifiée suivant la norme Pharmacopée Européenne 6.3 (01/2009:0008) et 9,027 Kg de chlorure d'aluminium hexahydraté (Panreac) ont été mélangés et agités pendant 10 minutes à l'aide d'un agitateur pneumatique à pâles éclipsables. Du fait de l'exothermicité de la réaction entre l'eau et le chlorure d'aluminium, toutes précautions utiles connues de l'homme du métier devront être utilisées.

15 g de bleu patenté (Spectracol) ont ensuite été progressivement ajoutés sous agitation dans le vortex du mélange précité et le mélange ainsi obtenu a été agité pendant 10 minutes à l'aide de l'agitateur pneumatique jusqu'à homogénéisation de la solution.

Toujours sous agitation, 8,4 Kg de glycérol (La Cooper) ont été ensuite ajoutés dans le vortex du mélange et l'agitation a été maintenue pendant 10 minutes supplémentaires à l'aide de l'agitateur pneumatique.

Etape 2: Dans la cuve d'un malaxeur à bras en Z muni d'un système de refroidissement (circulation d'eau froide dans la double enveloppe) la totalité du mélange préparé à l'étape 1, 14,4 Kg de fibres d'avoine et 11,4 Kg de carraghénanes de type viscarin® PC 389 ont été introduits puis pré-mélangés à une vitesse de 19 tr/m pendant 20 minutes.

Le malaxeur peut être optionnellement arrêté de manière à décoller la pâte des parois du malaxeur.

Le mélange ainsi préparé est ensuite agité pendant une durée supplémentaire de 40 minutes à la vitesse de 19 tr/m produisant ainsi un matériau d'insertion selon l'invention.

### EXEMPLES 2 à 5

En suivant un procédé analogue à celui décrit à l'exemple 1, on a préparé différents matériaux selon l'invention. La composition de chacun des matériaux réalisés est donnée en pourcentage en masse relativement à la masse totale du matériau dans le tableau I suivant.

**TABLEAU I**

| | **Exemple 2** | **Exemple 3** | **Exemple 4** | **Exemple 5** |
|---|---|---|---|---|
| Eau | 46,56 | 37 | 36,35 | 41 |
| Alcool polyvinylique | 2 | | | 1 |
| AlCl₃ | 9,44 | 9 | 9,06 | 9 |
| Glycérol | 10 | 10 | 15,71 | 15 |
| Carraqhénane ⁽¹⁾ | | 44 | 17,27 | 8 |
| Fibre d'avoine | | | 21,58 | 11 |
| Fibre de pois | 32 | | | 15 |
| Colorant | | | 0,03 | |
| Total | 100 | 100 | 100 | 100 |

| | | | | |
|---|---|---|---|---|
| ⁽¹⁾ Viscarin® PC 389 commercialisé par la société FMC BIOPOLYMERE. | | | | |

### EXEMPLE 6

### Mise en évidence des propriétés physico-chimiques du matériau selon l'invention.

### • Tests de dissolution

### - Protocole utilisé :

∘ Protocole suivi: Pharmacopée Européenne 2.9.3 "Essai de dissolution des formes solides"
∘ Type de dispositif utilisé: Appareil à palette Sotax
∘ Vitesse de rotation des pâles: 150 tours/min
∘ Température du bain thermostaté: 37°C
∘ volume de cuve = 700 ml
∘ Mode de prélèvement des échantillons du milieu de dissolution:
   - temps : t = 5 min, 10 min, 15 min, 30 min, 60 min
   - Méthode : prélèvement à l'aide d'une pipette en verre jaugée de précision classe A+
   - Volume de prélèvement = 50 ml
∘ Méthode d'analyse : Titrage complexométrique (suivant pharmacopée Europénne 2.5.11)

### - Résultats obtenus :

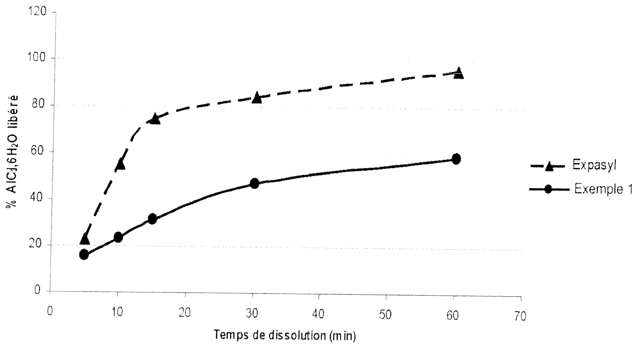

Les résultats de dissolution obtenus avec le matériau de l'Exemple 1 ont été comparés avec ceux obtenus avec le produit Expasyl®. Ces résultats, qui ont été reportés au tableau II et à la figure 1, montrent très clairement que les matériaux selon l'invention présentent une vitesse de dissolution caractérisée par une libération de l'agent astringent qui est significativement plus lente que dans le cas de l'Expasyl®.

**TABLEAU II**

| Temps (min) | 5 | 10 | 15 | 30 | 60 |
|---|---|---|---|---|---|
| Expasyl | 22,72 | 55,18 | 74,65 | 84,27 | 95,50 |
| Exemple 1 | 15,80 | 23,13 | 31,60 | 47,11 | 58,40 |

### • Test de désagrégation

- tests réalisés suivant la pharmacopée européenne 2.9.1 (désagrégation des comprimés et des capsules); essai A - comprimés et capsules de dimensions normales avec utilisation de disques cylindriques
- température d'immersion : 37°C
- milieu d'immersion : eau purifiée suivant la norme Pharmacopée Européenne 6.3 (01/2009:0008)
- le diamètre de l'échantillon : 10 mm
- épaisseur de l'échantillon : 4 mm
- résistance à la désagrégation : 5 heures

### • Tests d'extrusion et de cisaillement

Les essais d'extrusion ont été réalisés en utilisant une cartouche de forme cylindrique, de 30 mm de longueur et 4,5 mm de diamètre et ayant un convergent conique à l'extrémité de la filière présentant un rapport de réduction proche de 0,5 et d'angle 45°. Les essais d'extrusion ont été réalisés au moyen d'un analyseur de texture, pour deux vitesses imposées du piston, respectivement 1 et 3 mm/s.

Les valeurs de contrainte de friction et de seuil de cisaillement (kPa) ainsi que les coefficients de friction interne (kPa/mm) ont été déterminées pour un échantillon de formulations selon l'invention et sont reportés dans le tableau III suivant :

**TABLEAU III**

| | Exemple 2 | Exemple 3 | Exemple 4 |
|---|---|---|---|
| Contrainte de friction à 1 mm/s | 15,6 | 16,7 | 12,0 |
| Contrainte de friction à 3 mm/s | 18,3 | 25,9 | 10,9 |
| Seuil de cisaillement à 1 mm/s | 97,6 | 78,3 | 63,2 |
| Seuil de cisaillement à 3 mm/s | 118,5 | 99,5 | 80,2 |
| Coefficient de friction interne | 10,4 | 10,6 | 10,6 |

Les essais de cisaillement direct ont été réalisés au moyen d'un rhéomètre Brookfield Soft Solid Tester équipé d'une géométrie vanne à quatre pales de diamètre 5 mm et une hauteur de 10 mm. Ainsi l'influence de la vitesse de rotation de l'appareil sur le seuil statique (ou seuil de mise en écoulement) et le seuil dynamique a été étudié.

Ces tests ont permis de souligner :
- d'une part, que les matériaux de l'invention ont une friction régulière sur environ 90 % de la longueur du tube de la cartouche ; et
- d'autre part, que ces matériaux présentent un comportement rhéologique essentiellement plastique et un comportement tribologique de type glissant avec une friction seuil. Les seuils de plasticité et de friction semblent être influencés par la vitesse sans que l'effet puisse être apparenté à une viscosité.

## Revendications

1. Matériau d'insertion destiné à élargir le sillon gingival, **caractérisé en ce qu'**il est constitué d'une pâte hydrophile comprenant, exprimé en pourcentage en masse rapportée à la masse totale du matériau :
- entre 5 et 15 % d'un agent astringent;
- entre 25 et 50 % d'un agent de texture choisi parmi :
- les biopolymères hydrophiles ;
- les biopolymères hydrophiles associés à des fibres végétales ;
- les biopolymères hydrophiles associés à des fibres végétales et à de l'alcool polyvinylique ;
- les fibres végétales associées à de l'alcool polyvinylique ;
- entre 30 et 60 % d'eau ; et
- entre 0 et 20 % d'un agent humectant ;
- la quantité totale d'eau et d'agent humectant étant comprise entre 35 et 65 % en masse rapportée à la masse totale du matériau ;
- les biopolymères hydrophiles précités étant choisis dans le groupe constitué par les pectines ; les galactomannanes des graines de guar, de caroube, de tara ; le scléroglucane ; le xanthane et les carraghénanes, de préférence de type iota, et leurs mélanges ;
- les fibres végétales précitées étant choisies parmi les fibres de pois et les fibres d'avoine ;
- l'agent astringent étant choisi dans le groupe constitué des chlorures et sulfates de fer ou d'aluminium, du sulfate double d'aluminium et de potassium, et de leurs mélanges ;
- ledit matériau d'insertion présentant le profil de libération suivant de l'agent astringent, mesuré selon la méthode de dissolution décrite dans la Pharmacopée Européenne, 6^{ème} édition (Ph. Eur. 2.9.3 (01/2008)) à 150 t.p.m dans 700 ml d'eau purifiée à 37°C :
- moins de 20 % d'agent astringent libéré après 5 mn ;
- moins de 40 % d'agent astringent libéré après 10 mn ;
- moins de 50 % d'agent astringent libéré après 15 mn ;
- moins de 60 % d'agent astringent libéré après 30 mn ;
- moins de 80 % d'agent astringent libéré après 60 mn.

2. Matériau d'insertion selon la revendication 1, **caractérisé en ce que** la pâte hydrophile précitée comprend, exprimé en pourcentage en masse rapportée à la masse totale du matériau :
- entre 6 et 12 % d'un agent astringent;
- entre 30 et 45 % d'un agent de texture :
- entre 30 et 55 % d'eau ; et
- entre 5 et 18 % d'un agent humectant ;
la quantité totale d'eau et d'agent humectant étant comprise entre 40 et 60 % en masse rapportée à la masse totale du matériau.

3. Matériau d'insertion selon la revendication 1 ou 2, **caractérisé en ce qu'**il présente le profil de libération suivant de l'agent astringent :
- moins de 20 % d'agent astringent libéré après 5 mn ;
- moins de 30 % d'agent astringent libéré après 10 mn ;
- moins de 40 % d'agent astringent libéré après 15 mn ;
- moins de 55 % d'agent astringent libéré après 30 mn ;
- moins de 70 % d'agent astringent libéré après 60 mn.

4. Matériau d'insertion selon l'une quelconque des revendications 1 à 3, **caractérisé en ce que** les biopolymères hydrophiles précités sont choisis parmi les carraghénanes, de préférence de type iota.

5. Matériau d'insertion selon l'une quelconque des revendications 1 à 4, **caractérisé en ce que** les fibres végétales précitées sont choisies parmi les fibres de pois et les mélanges de fibres de pois et de fibres d'avoine.

6. Matériau d'insertion selon l'une quelconque des revendications 1 à 5, **caractérisé en ce que** l'agent astringent est le chlorure de fer.

7. Matériau d'insertion selon l'une quelconque des revendications 1 à 5, **caractérisé en ce que** l'agent astringent précité est le chlorure d'aluminium.

8. Matériau d'insertion selon l'une quelconque des revendications 1 à 7, **caractérisé en ce que** l'agent humectant précité est choisi dans le groupe constitué par le glycérol, le polyéthylèneglycol, le sorbitol et leurs mélanges.

9. Matériau d'insertion selon la revendication 8, **caractérisé en ce que** l'agent humectant précité est le glycérol.

10. Matériau d'insertion selon l'une quelconque des revendications 1 à 9, **caractérisé en ce qu'**il contient en outre jusqu'à 1% en masse, et de préférence de 0,01 à 0,5 % en masse, d'au moins un additif choisi parmi les colorants et les arômes, de préférence de qualité alimentaire.

11. Matériau d'insertion selon l'une quelconque des revendications 1 à 10, **caractérisé en ce qu'**il contient en outre jusqu'à 10 % en masse, et de préférence de 2 à 10 % en masse, d'au moins une substance active choisie parmi les anesthésiques locaux, de préférence la lidocaïne.

12. Matériau d'insertion selon l'une quelconque des revendications 1 à 10, **caractérisé en ce qu'**il présente l'une au moins des caractéristiques suivantes :
- un comportement tribologique de type glissant avec une friction seuil, de préférence comprise entre 10 et 60 kPa ;
- un seuil de cisaillement initial compris entre 30 et 350 kPa ;
- un module de Young compris entre 65 et 200 kPa.

## Patentansprüche

1. Einsetzmaterial zur Erweiterung der Zahnfleischfurche, **dadurch gekennzeichnet, dass** es aus einer hydrophilen Paste besteht, die, ausgedrückt in Masseprozent bezogen auf die Gesamtmasse des Materials, umfasst:
- zwischen 5 und 15 % eines adstringierenden Mittels,
- zwischen 25 und 50 % eines Texturmittels, ausgewählt aus:
- hydrophilen Biopolymeren,
- hydrophilen Biopolymeren, die mit Pflanzenfasern assoziiert sind,
- hydrophilen Biopolymeren, die mit Pflanzenfasern und mit Polyvinylalkohol assoziiert sind,
- Pflanzenfasern, die mit Polyvinylalkohol assoziiert sind,
- zwischen 30 und 60 % Wasser und
- zwischen 0 und 20 % eines Feuchthaltemittels,
- wobei die Gesamtmenge an Wasser und Feuchthaltemittel zwischen 35 und 65 Masse-% bezogen auf die Gesamtmasse des Materials beträgt,
- wobei die vorgenannten hydrophilen Biopolymere aus der Gruppe bestehend aus Pektinen, Galactomannanen von Guar-, Johannisbrot-, Tarakernen, Scleroglucan, Xanthan und Carrageenen, vorzugsweise vom iota-Typ, sowie deren Mischungen ausgewählt sind,
- wobei die vorgenannten Pflanzenfasern aus Erbsenfasern und Haferfasern ausgewählt sind,
- wobei das adstringierende Mittel aus der Gruppe bestehend aus Eisen- oder Aluminiumchloriden und -sulfaten, Aluminium- und Kalium-Doppelsulfat sowie deren Mischungen ausgewählt ist,
- wobei das Einsetzmaterial das folgende Freisetzungsprofil des adstringierenden Mittels, gemessen nach der in dem Europäischen Arzneibuch, 6. Ausgabe (Ph. Eur. 2.9.3 (01/2008)) beschriebenen Auflösungsmethode, bei 150 U/Min in 700 ml gereinigtem Wasser, bei 37 °C, aufweist:
- weniger als 20 % adstringierendes Mittel nach 5 Min. freigesetzt,
- weniger als 40 % adstringierendes Mittel nach 10 Min. freigesetzt,
- weniger als 50 % adstringierendes Mittel nach 15 Min. freigesetzt,
- weniger als 60 % adstringierendes Mittel nach 30 Min. freigesetzt,
- weniger als 80 % adstringierendes Mittel nach 60 Min. freigesetzt.

2. Einsetzmaterial nach Anspruch 1, **dadurch gekennzeichnet, dass** die vorgenannte hydrophile Paste, ausgedrückt in Masseprozent bezogen auf die Gesamtmasse des Materials, umfasst:
- zwischen 6 und 12 % eines adstringierenden Mittels,
- zwischen 30 und 45 % eines Texturmittels,
- zwischen 30 und 55 % Wasser und
- zwischen 5 und 18 % eines Feuchthaltemittels,
wobei die Gesamtmenge an Wasser und Feuchthaltemittel zwischen 40 und 60 Masse-% bezogen auf die Gesamtmasse des Materials beträgt.

3. Einsetzmaterial nach Anspruch 1 oder 2, **dadurch gekennzeichnet, dass** es das folgende Freisetzungsprofil des adstringierenden Mittels aufweist:
- weniger als 20 % adstringierendes Mittel nach 5 Min. freigesetzt,
- weniger als 30 % adstringierendes Mittel nach 10 Min. freigesetzt,
- weniger als 40 % adstringierendes Mittel nach 15 Min. freigesetzt,
- weniger als 55 % adstringierendes Mittel nach 30 Min. freigesetzt,
- weniger als 70 % adstringierendes Mittel nach 60 Min. freigesetzt.

4. Einsetzmaterial nach einem der Ansprüche 1 bis 3, **dadurch gekennzeichnet, dass** die vorgenannten hydrophilen Biopolymere aus den Carrageenen, vorzugsweise vom iota-Typ ausgewählt sind.

5. Einsetzmaterial nach einem der Ansprüche 1 bis 4, **dadurch gekennzeichnet, dass** die vorgenannten Pflanzenfasern aus den Erbsenfasern und den Mischungen aus Erbsenfasern und Haferfasern ausgewählt sind.

6. Einsetzmaterial nach einem der Ansprüche 1 bis 5, **dadurch gekennzeichnet, dass** das adstringierende Mittel Eisenchlorid ist.

7. Einsetzmaterial nach einem der Ansprüche 1 bis 5, **dadurch gekennzeichnet, dass** das vorgenannte adstringierende Mittel Aluminiumchlorid ist.

8. Einsetzmaterial nach einem der Ansprüche 1 bis 7, **dadurch gekennzeichnet, dass** das vorgenannte Feuchthaltemittel aus der Gruppe bestehend aus Glycerin, Polyethylenglykol, Sorbitol und deren Mischungen ausgewählt ist.

9. Einsetzmaterial nach Anspruch 8, **dadurch gekennzeichnet, dass** das Feuchthaltemittel Glycerin ist.

10. Einsetzmaterial nach einem der Ansprüche 1 bis 9, **dadurch gekennzeichnet, dass** es ferner bis zu 1 Masse-% und vorzugsweise 0,01 bis 0,5 Masse-% wenigstens eines Additivs enthält, das aus Farbstoffen und Aromen, vorzugsweise mit Lebensmittelqualität, ausgewählt ist.

11. Einsetzmaterial nach einem der Ansprüche 1 bis 10, **dadurch gekennzeichnet, dass** es ferner bis zu 10 Masse-% und vorzugsweise 2 bis 10 Masse-% wenigstens eines Wirkstoffs enthält, der aus den Lokalanästhetika, vorzugsweise Lidocain, ausgewählt ist.

12. Einsetzmaterial nach einem der Ansprüche 1 bis 10, **dadurch gekennzeichnet, dass** es wenigstens eines der folgenden Merkmale aufweist:
- ein tribologisches Verhalten vom Typ gleitend mit einer Schwellenreibung, vorzugsweise im Bereich zwischen 10 et 60 kPa,
- einen anfänglichen Scherschwellwert im Bereich zwischen 30 und 350 kPa,
- einen Elastzitätsmodul im Bereich zwischen 65 et 200 kPa.

## Claims

1. An insertion material intended for widening the gingival crevice, **characterized in that** it consists of a hydrophilic paste comprising, expressed as percentage by weight relative to the total weight of the material:
- between 5 and 15% of an astringent;
- between 25 and 50% of a texturizing agent chosen from:
- hydrophilic biopolymers;
- hydrophilic biopolymers combined with plant fibers;
- hydrophilic biopolymers combined with plant fibers and with polyvinyl alcohol;
- plant fibers combined with polyvinyl alcohol;
- between 30 and 60% of water; and
- between 0 and 20% of a humectant;
- the total amount of water and humectant being between 35 and 65% by weight relative to the total weight of the material;
- the abovementioned hydrophilic biopolymers being chosen from the group consisting of pectins; galactomannans of guar, locust bean or tara seeds; scleroglucan; xanthan and carrageenans, preferably of iota type, and mixtures thereof;
- the abovementioned plant fibers being chosen from pea fibers and oat fibers;
- the astringent being chosen from the group consisting of iron or aluminum chlorides and sulfates, potassium aluminum sulfate, and mixtures thereof;
- said insertion material having the following release profile for the astringent, measured according to the dissolution method described in the European Pharmacopoeia, 6th edition (Ph. Eur. 2.9.3 (01/2008)) at 150 rpm in 700 ml of purified water at 37°C:
- less than 20% of astringent released after 5 min;
- less than 40% of astringent released after 10 min;
- less than 50% of astringent released after 15 min;
- less than 60% of astringent released after 30 min;
- less than 80% of astringent released after 60 min.

2. The insertion material as claimed in claim 1, **characterized in that** the abovementioned hydrophilic paste comprises, expressed as percentage by weight relative to the total weight of the material:
- between 6 and 12% of an astringent;
- between 30 and 45% of a texturizing agent;
- between 30 and 55% of water; and
- between 5 and 18% of a humectant;
the total amount of water and humectant being between 40 and 60% by weight relative to the total weight of the material.

3. The insertion material as claimed in claim 1 or 2, **characterized in that** it has the following release profile for the astringent:
- less than 20% of astringent released after 5 min;
- less than 30% of astringent released after 10 min;
- less than 40% of astringent released after 15 min;
- less than 55% of astringent released after 30 min;
- less than 70% of astringent released after 60 min.

4. The insertion material as claimed in any one of claims 1 to 3, **characterized in that** the abovementioned hydrophilic biopolymers are chosen from carrageenans, preferably of iota type.

5. The insertion material as claimed in any one of claims 1 to 4, **characterized in that** the abovementioned plant fibers are chosen from pea fibers and mixtures of pea fibers and oat fibers.

6. The insertion material as claimed in any one of claims 1 to 5, **characterized in that** the astringent is iron chloride.

7. The insertion material as claimed in any one of claims 1 to 5, **characterized in that** the abovementioned astringent is aluminum chloride.

8. The insertion material as claimed in any one of claims 1 to 7, **characterized in that** the abovementioned humectant is chosen from the group consisting of glycerol, polyethylene glycol and sorbitol, and mixtures thereof.

9. The insertion material as claimed in claim 8, **characterized in that** the abovementioned humectant is glycerol.

10. The insertion material as claimed in any one of claims 1 to 9, **characterized in that** it also contains up to 1% by weight, and preferably from 0.01 to 0.5% by weight, of at least one additive chosen from dyes and flavorings, preferably of food grade.

11. The insertion material as claimed in any one of claims 1 to 10, **characterized in that** it also contains up to 10% by weight, and preferably from 2 to 10% by weight, of at least one active substance chosen from local anesthetics, preferably lidocaine.

12. The insertion material as claimed in any one of claims 1 to 10, **characterized in that** it has at least one of the following characteristics:
- a sliding tribological behavior with a threshold friction, preferably between 10 and 60 kPa;
- an initial shear threshold of between 30 and 350 kPa;
- a Young's modulus of between 65 and 200 kPa.
